Europäisches Patentamt

European Patent Office

Office européen des brevets

(11) Publication number: **0 057 177**
**B1**

(12) # EUROPEAN PATENT SPECIFICATION

(45) Date of publication of patent specification: **24.10.84**

(21) Application number: **80901673.6**

(22) Date of filing: **12.08.80**

(86) International application number:
**PCT/SE80/00202**

(87) International publication number:
**WO 82/00698 04.03.82 Gazette 82/07**

(51) Int. Cl.³: **F 16 L 21/02,** F 16 L 25/00,
F 16 L 37/28, A 61 M 1/03,
A 61 M 5/31

(54) **ARRANGEMENT IN A STERILIZING COUPLING.**

(43) Date of publication of application:
**11.08.82 Bulletin 82/32**

(45) Publication of the grant of the patent:
**24.10.84 Bulletin 84/43**

(84) Designated Contracting States:
**AT CH DE FR GB LI NL SE**

(56) References cited:
**WO-A-80/01310**
**WO-A-80/01507**
**GB-A-1 453 634**
**GB-A-2 008 705**
**SE-B- 314 259**
**US-A-3 201 148**

(73) Proprietor: **SVENSSON, Jan Axel**
**Solhemsgatan 12**
**S-561 35 Huskvarna (SE)**

(72) Inventor: **SVENSSON, Jan Axel**
**Solhemsgatan 12**
**S-561 35 Huskvarna (SE)**

(74) Representative: **Ström, Tore et al**
**Ström & Gulliksson AB Studentgatan 1 P.O. Box 4188**
**S-203 13 Malmö (SE)**

EP 0 057 177 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to an arrangement in a sterilizing coupling, more precisely to a coupling of the type described and illustrated in the International Patent Publication WO—A—80/01507.

Thus, in conjunctoin with Fig. 11 of the drawings in said International Publication, a coupling is shown in which a sterilizing action is obtained by utilizing a sealing ring prepared with a disinfectant, which gives off disinfectant to the coupling component which comes into contact with the ring during engagement. When using couplings of this type e.g. in conjunction with dialysis procedures the demands for sterility are extremely great.

The object of the present invention is thus to provide a coupling of the type referred to in WO—A—80/01507, which is self-sterilizing during use. This object is achieved in accordance with the present invention in that the coupling has obtained the characteristics specified in claim 1.

To illustrate the invention this will be described in more detail in the following with reference to the accompanying drawings, in which

Fig. 1 shows a vertical section of the coupling in accordance with the invention before the coupling element is connected to the coupling housing;

Fig. 2 shows the coupling components according to Fig. 1 during initial engagement;

Fig. 3 shows the coupling in the engaged position which permits through-flow; and

Fig. 4 illustrates the coupling in a position wherein the coupling element is being disengaged from the coupling housing.

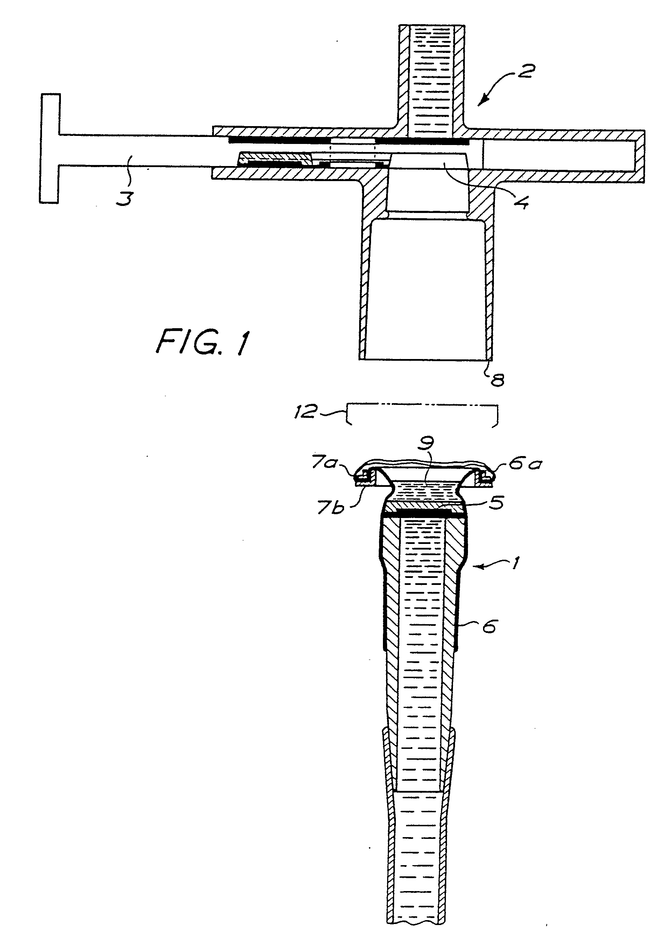

Fig. 1 shows the coupling element 1 and coupling housing 2 in a disengaged position. The coupling housing has a movable slide 3 in which at least one recess 4 is arranged for accommodating a cover, and the coupling element 1 has a cover 5 which is received for displacement in a groove. The more detailed design of the coupling is disclosed in WO—A—80/01507.

The new feature of the coupling in accordance with the present invention is the arrangement of a casing 6 which is located around the coupling element 1. The casing 6 is preferably made from an elastic material, e.g. latex rubber, and extends up and over the cover 5. Two ring-shaped elements 7a and 7b with L-shaped cross-section are arranged on both sides of the casing adjacent to the free end 6a of the casing 6. Instead of the two ring-shaped elements 7a and 7b a reinforcement can be moulded into the casing 6, which—similar to the elements 7a and 7b—is pushed in and sealingly rests against the internal peripheral surface of an abutment 8.

Inside the upper section of the casing 6 there is a disinfectant, e.g. spirit, which forms a barrier in respect of bacteria and also has a sterilizing effect on the coupling components during their engagement.

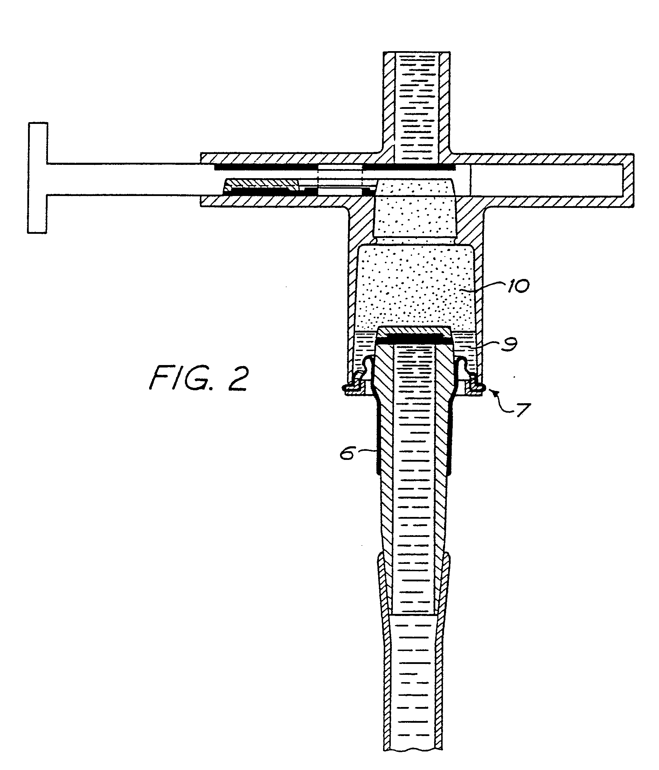

Fig. 2 illustrates the coupling components during initial engagement, whereby the disinfectant is present in the cavity 10. By making the casing 6 elastic, this will expand somewhat during the continued insertion of the coupling element 1 into the coupling housing 2.

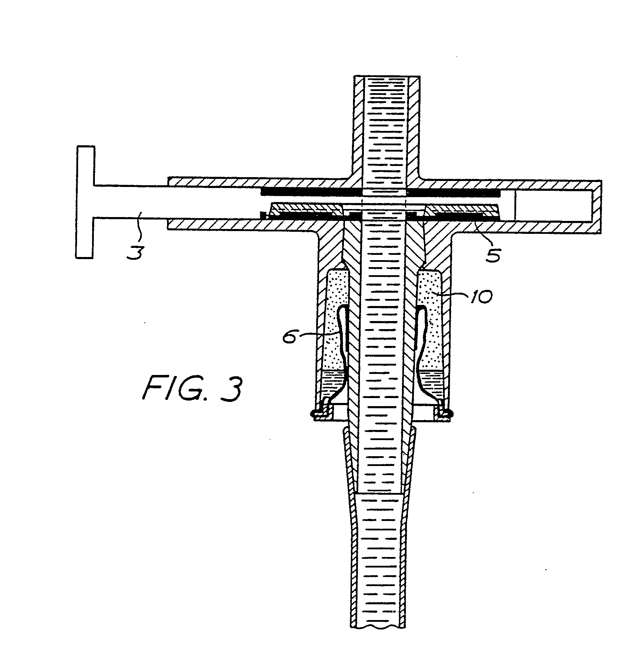

Fig. 3 illustrates the two coupling components 1 and 2 in an engaged and locked position, said locking being achieved by displacement of the slide 3, i.e. the coupling permits free passage through both coupling components. However, external locking can also be provided between the coupling element 1 and the coupling housing 2, i.e. the coupling components should be capable of being locked in an engaged position without a free passage necessarily existing between the coupling components. This facilitates operation of the coupling by preventing the casing 6 pushing the coupling components apart. Such a locking device between the coupling components can for example comprise a spring lock, etc.

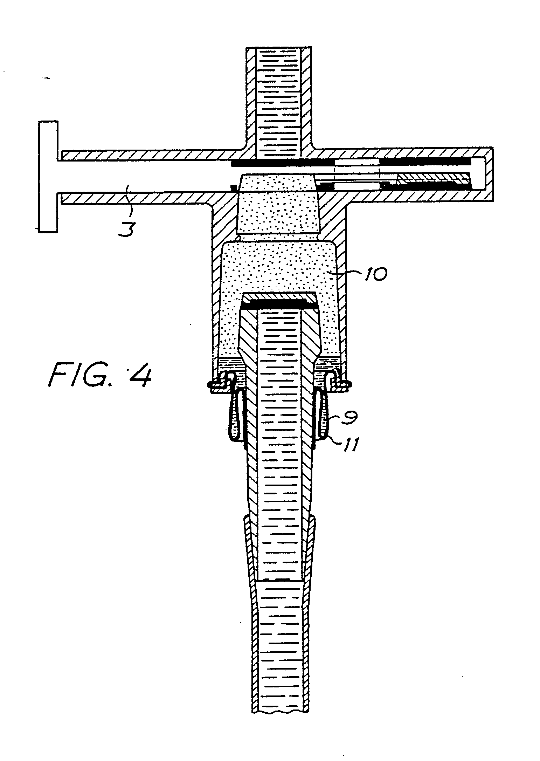

When separating the coupling components (Fig. 4), the disinfectant 9 collects in the folded portion 11 of the casing, which means that a thin film of disinfectant will be present between the outside wall of the coupling element 1 and the casing 6 when the coupling element is removed completely from the coupling housing 2.

The coupling element 1 with the casing 6 arranged thereon can be supplied complete with liquid disinfectant, which means that a cover 12 (Fig. 1) of a suitable type has to be provided at the free end 6a of the casing 6.

Disinfectant is replenished as required in casing 6.

## Claims

1. Arrangement in a coupling comprising a coupling element (1) closed at one end by means of a slide valve (5) and a coupling housing (2) to be connected therewith and having a socket into which the coupling element can be introduced, characterized in that a thin flexible casing (6) which surrounds at least a part of the coupling element (1) and projects axially from the closed end thereof to define together with the coupling element a cavity for accommodating a distinfection agent (9), at the projecting end thereof is capable of being connected essentially fluid-tight to the socket to be pulled backwards over the coupling element (1) at the introduction thereof into the socket in order that during introduction into the socket the coupling element (1) shall be passed through the disinfection fluid.

2. Arrangement according to claim 1, characterized by a locking device for disengagable holding of the coupling element (1) and the coupling housing (2) with the coupling element fully introduced into the socket.

3. Arrangement according to claim 1, characterized in that a cover (12) is provided at the free end (6a) of the casing (6).

## Revendications

1. Arrangement dans un raccordement comprenant un élément (1) de raccordement fermé à une extrémité au moyen d'une vanne (5) à tiroir, et un logement (2) de raccordement destiné à le recevoir et présentant un manchon où l'élément de raccordement peut être introduit, caractérisé en ce qu'une fine enveloppe flexible (6), qui entoure une partie au moins de l'élément (1) de raccordement et se prolonge axialement depuis l'extrémité fermée de façon à définir avec cet élément une cavité destinée à recevoir un agent (9) de désinfection, peut être raccordée à l'extrémité faisant saillié au manchon de façon essentiellement étanché afin d'être retirée vers l'arrière par dessus l'élément (1) de raccordement en sorte que, durant son introductoin dans le manchon, l'élément (1) de raccordement va passer à travers le fluide de désinfection.

2. Arrangement selon la revendication 1, caractérisé en ce qu'il comprend un organe de verrouillage pour maintenir de façon désengageable l'élément (1) de raccordement et le logement (2) de raccordement quand l'élément de raccordement est complètement introduit dans le manchon.

3. Arrangement selon la revendication 1, caractérisé en ce qu'il comprend un couvercle (12) à l'extrémité (6a) libre de l'enveloppe (6).

## Patentansprüche

1. Anordnung in einer Kupplung, bestehend aus einem an einem Ende mit Hilfe eines Schiebers (5) geschlossenen Kupplungselement (1) und aus einem hiermit zu verbindenden Kupplungselement (2) mit einem Stutzen, in den das Kupplungselement einsetzbar ist, dadurch gekennzeichnet, dass eine dünne flexible Hülle (6), welche zumindest einen Teil des Kupplungselementes (1) umgibt und von dessen geschlossenem Ende axial vorragt, um gemeinsam mit dem Kupplungselement einen Hohlraum zur Aufnahme eines Desinfektionsmittels (9) zu begrenzen, an ihrem vorragenden Ende zum im wesentlichen fluid-dichten Anschluss an den Stutzen aus-gebildet ist, um rückwärts über das Kupplungselement (1) an dessen Einsetzung in den Stutzen gezogen zu werden, damit das Kupplungselement (1) während des Einsetzens in den Stutzen durch das Desinfektionsmittel geführt werden soll.

2. Anordnung nach Anspruch 1, gekennzeichnet durch eine Sperrvorrichtung zum lösbaren Festhalten des Kupplungselementes (1) sowie des Kupplungsgehäuses (2) bei vollständig in den Stutzen eingesetztem Kupplungselement.

3. Anordnung nach Anspruch 1, dadurch gekennzeichnet, dass am freien Ende (6a) der Hülle (6) ein Verschluss (12) vorgesehen ist.

0 057 177

*FIG. 1*

1

FIG. 2

FIG. 3

FIG. 4